(19) Europäisches Patentamt
European Patent Office
Office européen des brevets

(11) **EP 2 929 857 A1**

(12) **EUROPÄISCHE PATENTANMELDUNG**

(43) Veröffentlichungstag:
**14.10.2015  Patentblatt 2015/42**

(51) Int Cl.:
*A61C 17/06* (2006.01)     *A61M 1/00* (2006.01)

(21) Anmeldenummer: **14164356.9**

(22) Anmeldetag: **11.04.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Wegold Edelmetalle GmbH**
**90530 Wendelstein (DE)**

(72) Erfinder: **Klück, Stefan**
**90530 Wendelstein (DE)**

(74) Vertreter: **Stork Bamberger**
**Patentanwälte**
**Postfach 73 04 66**
**22124 Hamburg (DE)**

(54) **Filter zum Einfügen in eine Absaugeinrichtung**

(57)     Ein Filter 1, ausgebildet und eingerichtet zum Einfügen in eine Absaugeinrichtung, umfasst ein Filtergewebe 15 und einen Filterkörper zur Aufnahme des Filtergewebes 15, wobei der Filterkörper ein als Hohlkörper mit mindestens zwei Öffnungen ausgebildetes Filtergehäuse 2 mit einem Schlauchanschlussabschnitt 3 an einer ersten Gehäuseöffnung und mit einem Eingangsabschnitt 5 an einer zweiten Gehäuseöffnung umfasst. Ferner ist das Filtergewebe 15 sackförmig ausgebildet, die offene Fläche des Filtergewebes 15 beträgt mindestens das Doppelte der Fläche des Querschnitts des äußeren Endes des Schlauchanschlussabschnitts 3 beträgt, das Filtergewebe 15 umfasst Abschnitte mit einer Maschenweite w von 10 bis 80 $\mu$m umfasst und der Fadendurchmesser d der Fäden dieser Abschnitte des Filtergewebes 15 beträgt von 10 bis 80 $\mu$m.

fig. 4

EP 2 929 857 A1

Printed by Jouve, 75001 PARIS (FR)

**Beschreibung**

Die vorliegende Erfindung betrifft einen Filter, ausgebildet und eingerichtet zum Einfügen in eine Absaugeinrichtung, umfassend ein Filtergewebe und einen Filterkörper zur Aufnahme des Filtergewebes, wobei der Filterkörper ein als Hohlkörper mit zwei Öffnungen ausgebildetes Filtergehäuse mit einem Schlauchanschlussabschnitt an einer ersten Gehäuseöffnung und mit einem Eingangsabschnitt an einer zweiten Gehäuseöffnung umfasst.

Insbesondere aus der zahnärztlichen Praxis sind Absaugeinrichtungen allgemein bekannt. Absaugeinrichtungen bestehen aus einem Vakuumerzeuger und einem Rohr-/Schlauchsystem. Solche Absaugeinrichtungen dienen dazu, bei Schleif- und Bohrarbeiten an den Zähnen eines Patienten entstehende Partikel aus dem Behandlungsbereich im Mundraum zu entfernen. Dies ist zum einen erforderlich, um dem Behandelnden eine ausreichende Sicht auf den Behandlungsbereich zu gewährleisten, zum anderen um weitestgehend zu verhindern, dass der Patient die entstandenen Partikel verschluckt. Bei chirurgischen Eingriffen kann es darüber hinaus von Interesse sein, körpereigenes Gewebe zwecks späterer Verwendung aufzufangen. Die Partikel bilden zusammen mit dem Speichel des Patienten und sonstigen im Mund enthaltenen Flüssigkeiten wie z.B. Spülflüssigkeit die Absauglösung. Diese Absauglösungen dürfen nicht unbehandelt dem Abwasser zugeführt werden, da sie giftige Stoffe wie beispielsweise Amalgam enthalten können.

Daher sind in den Absaugeinrichtungen sogenannte Amalgamabscheider eingebaut, um das Abwasser von Quecksilberbelastungen zu reinigen. Andere Schwebstoffe und Partikel setzen sich jedoch ebenfalls im Amalgamabscheider ab und erhöhen dessen Wartungsaufwand, da die Filter häufiger gewechselt werden müssen, sich das Wartungsintervall also verkürzt. In zyklischen Abständen werden die sich in den Amalgamabscheidern befindlichen Sammelbehälter mit den abgeschiedenen Stoffen entnommen und einem Recycling- oder Entsorgungsprozess zugeführt. Bei Amalgamabscheidern sind unterschiedliche Systeme zum Abscheiden bekannt. Sie beruhen auf dem Zyklonverfahren oder auf dem Gravitationsverfahren oder stellen eine Kombination aus beiden dar. Ein Beispiel für einen Schwerkraftabscheider ist in der DE G 83 14 829 beschrieben.

Neben den unerwünschten Amalgamen werden insbesondere beim Entfernen von Kronen und Brücken auch andere Stoffe wie zum Beispiel Edelmetalle als Schleifstaub aufgesaugt. Auch werden regelmäßig versehentlich größere Objekte eingesaugt, z.B. Inlays oder Kronen. Aus diesem Grund enthalten viele Absauganlagen zusätzliche Grobfilter, mit Hilfe derer die größeren Objekte zurückerhalten werden können. Allerdings ist deren Handhabung häufig sehr umständlich und die Wiederbeschaffung der versehentlich eingesaugten Objekte nur sehr mühsam möglich.

Kleinere Partikel von Edelmetallen gelangen zusammen mit den unerwünschten Amalgamrückständen in den Amalgamfilter. Wenn dessen Filterbehälter ausgewechselt werden, wird somit nicht nur das unerwünschte Amalgam entsorgt, sondern auch weitere, teilweise wertvolle Partikel. Das Separieren der verschiedenen Partikel im Amalgamfilter ist nur mit unverhältnismäßig großem Aufwand möglich.

Es ist daher wünschenswert, eine Möglichkeit zum Separieren gewünschter Partikel von den unerwünschten Partikeln vorzusehen, die verhindert, dass Edelmetallpartikel oder andere Partikel, die für eine weitere Verwendung von Interesse sind, in den Amalgamabscheider gelangen und als Abfall entsorgt werden.

Eine automatische Trennung von Amalgam und anderen Partikeln ist bislang nicht möglich. Es gibt jedoch einige Patente, die sich mit der Thematik des manuellen Herausfilterns gewünschter Partikel aus dentalen Absauglösungen beschäftigen. Zu den gewünschten Partikeln gehören Knochenmaterial, Strahlsalze von Zahnreinigung und insbesondere auch Edelmetallstäube.

Für die Rückgewinnung von Knochenmaterial ist ein unter der Bezeichnung "BoneTrap™" am Markt erhältlicher Filter bekannt. Dabei handelt es sich um einen zylindrischen multi-perforierten Filter, der mit dem sterilen Schlauch des zahnärztlichen Saugers verbunden wird. Der Filter weist verhältnismäßig große Perforationen auf, um einen hinreichenden Durchfluss zu gewährleisten und ein Verstopfen zu verhindern.

Die DE 10 2009 034 143 A offenbart eine Absaugvorrichtung mit zwei verschiedenen Partikeltrenneinrichtungen und einer Umschaltvorrichtung. Die Absaugvorrichtung dient dazu, edelmetallhaltige Partikel und Stäube aus Absaugflüssigkeiten zu gewinnen. Mittels dieser Umschaltvorrichtung kann zwischen den beiden Partikeltrenneinrichtungen hin und her geschaltet werden. Wenn aus dem Mundraum Absaugflüssigkeiten entfernt werden, die im Wesentlichen unerwünschte Partikel enthalten, wird mittels der Umschaltvorrichtung der Fluss der Absaugflüssigkeit in eine erste Partikeltrenneinrichtung geleitet, die einen Filterbehälter umfasst, in dem zu entsorgende Partikel gesammelt und aus der Absaugflüssigkeit entfernt werden. Wenn jedoch ein Absaugen von erwünschten Partikeln bzw. von Absaugflüssigkeit mit solchen erwünschten Partikeln erfolgen soll, dann wird mit der Umschaltvorrichtung der Fluss zu einer zweiten Partikeltrenneinrichtung geleitet, mittels derer die gewünschten Partikel wie insbesondere Edelmetallpartikel herausgefiltert werden. Diese zweite Partikeltrenneinrichtung ist derart ausgebildet, dass sie im Mund des Patienten angeordnet werden kann.

Eine weitere Partikeltrenneinrichtung ist in der DE 541 402 beschrieben. Diese Schrift offenbart ein rohrförmiges, aus lösbaren Einzelteilen bestehendes Anschlussstück für Leitungen zum Absaugen von Abfällen und Flüssigkeiten bei zahnärztlichen Arbeiten mit querliegenden Filtereinlagen mit gegeneinander versetzten Durchtrittsöffnungen für die Saugluft und auswechselbaren Hülsen aus rauem, die Flüssigkeiten aufsaugendem Stoff in einzelnen Teilen des Anschlussstückes, wobei die Filtereinlagen als gelochte Säcke ausgebildet sein können, die durch Spreizfedern offen gehalten werden. Aufgrund der erforderlichen Durchtrittsöffnungen sind mindestens zwei Filtereinlagen notwendig, bei denen die Durchtrittsöffnungen zueinander versetzt sind, um weitest möglich zu verhindern, dass Partikel durch diese Durchtrittsöffnungen in den Flüssigkeiten mitgeführt und nicht herausgefiltert werden. Trotzdem kann nicht verhindert werden, dass auch größere Partikel an den Filtereinlagen vorbeigelangen und in der Flüssigkeit verbleiben.

Keine der Trenn- oder Filtereinrichtungen des Standes der Technik zum Abtrennen von Edelmetallpartikeln bzw. -stäuben hat sich am Markt durchsetzen können. Dies liegt darin begründet, dass alle Filtereinrichtungen des Standes der Technik mit einem oder mehreren Nachteilen behaftet sind. Diese Nachteile haben ihre Ursache zumeist in der Saugleistung der Absaugeinrichtung. Die Saugleistung muss hinreichend stark sein, um ein sicheres Einsaugen auch größerer und schwererer Partikel zu gewährleisten. Die Saugleistung wird allerdings aufgrund der eingeschalteten Filter stark verringert. Wie stark die Saugleistung reduziert wird, hängt von der Ausbildung des Filters ab. Üblicherweise nimmt die Saugleistung umso stärker ab, je feiner der verwendete Filter ist. Dies hat zur Folge, dass man einen Kompromiss zwischen der Saugleistung und der Größe der herauszufilternden Partikel eingehen muss. Soll die Saugleistung ausreichend stark bleiben, sind die Filter entsprechend grobporiger zu wählen, wodurch feine Artikel jedoch nicht herausgefiltert werden können.

Alternativ werden Filter mit einem sehr großen Durchmesser verwendet. Ein solcher Filter wird unter der Bezeichnung "HD-Gold-Filter" von der Firma Hentschel-Dental e.K. vertrieben. Der Filter besteht aus einem sich konisch erweiternden und wieder verjüngenden Kunststoffgehäuse, dessen Form einem Rotationsrhombus angenähert ist. An der Stelle des größten Durchmessers ist in dem Kunststoffgehäuse eine Edelstahlnetzeinlage eingesetzt. Auf diese Netzeinlage wird ein Filtervlies aufgebracht, auf dem sich die Edelmetall-, in diesem Fall insbesondere Goldpartikel sammeln. Ein solcher Filter ist jedoch sehr groß und unhandlich und daher für den Gebrauch in der Praxis nur bedingt geeignet, denn der Filter ist vorzugsweise als Einsatz nahe dem Mundstück der Absaugeinrichtung angebracht, um ihn schnell und einfach während der Behandlung einsetzen zu können. Ein großer Einsatz nahe dem Mundstück schränkt jedoch die Beweglichkeit des oberen Teils der Saugeinrichtung stark ein. Eine solche Beweglichkeit ist aber gewünscht, um einfach mit dem Mundstück hantieren zu können und frei zu sein in der Positionierung des Mundstückes im Mundraum des Patienten.

Der Erfindung lag daher die Aufgabe zugrunde, einen Filter zu schaffen, der auf einfache Weise in eine zahnärztliche Absaugeinrichtung integriert werden kann, der leicht auszuwechseln ist, der ermöglicht, dass eine hinreichende Absaugleistung der Absaugeinrichtung gewährleistet ist, der die Handhabung der Absaugeinrichtung und insbesondere deren Mundstück nicht beeinträchtigt und der einen größtmöglichen Anteil der in einer Absauglösung enthaltenen Edelmetallpartikel herausfiltert und zurückhält. Filter für zahnärztliche Absaugvorrichtungen werden häufig als Dentalfilter bezeichnet.

Erfindungsgemäß wird die Aufgabe durch einen Filter der eingangs beschriebenen Art gelöst, bei dem das Filtergewebe sackförmig ausgebildet ist, die offene Fläche des Filtergewebes mindestens das Doppelte der Fläche des Querschnitts des äußeren Endes des Schlauchanschlussabschnitts beträgt, das Filtergewebe Abschnitte mit einer Maschenweite w von 10 bis 80 $\mu$m umfasst und der Fadendurchmesser d der Fäden dieser Abschnitte des Filtergewebes von 10 bis 80 $\mu$m beträgt.

Ein solcher erfindungsgemäßer Filter ist sehr leicht in eine zahnärztliche Absaugeinrichtung integrierbar. Vorzugsweise wird er zwischen dem Mundstück und dem zugehörigen Schlauchansatz eingesetzt. Damit ist er leicht zugänglich und kann leicht ausgewechselt werden. Auch wenn beim Einsaugen versehentlich unbeabsichtigt z.B. ganze Brücken oder Kronen eingesaugt werden, bleiben diese im Filter hängen und können leicht herausgenommen werden. Der Filter wird nur im Bedarfsfall in die Absaugeinrichtung eingesetzt. Dies ist mit wenigen Handgriffen möglich. Durch die kompakte Gestaltung des Filters beeinträchtigt dieser die Arbeit des Zahnarztes und seiner Helfer nicht. Das Mundstück der Absaugeinrichtung kann mit eingesetztem Filter in gleicher Weise gehandhabt werden wie ohne Filter. Die Saugleistung der Absaugeinrichtung ist trotz des Filters ausreichend groß. Wenn eine größere Menge Edelmetallpartikel im Filtersack aufgenommen ist, ist dies durch einen Abfall der Saugleistung erkennbar. Der Filter wird dann ausgetauscht, was aufgrund der einfachen Handhabbarkeit und guten Zugänglichkeit leicht möglich ist.

Aufgrund der sackförmigen Ausbildung des Filtergewebes kann dieses aus dem Filterkörper entnommen und weiter gehandhabt und zum Beispiel dem Recycling zugeführt werden, ohne dass Edelmetallpartikel verlorengehen, wie dies bei scheibenförmiger Ausbildung des Filtergewebes oft der Fall ist. Die Handhabung des mit Edelmetallpartikeln gefüllten Filtergewebes ist somit ebenfalls stark verbessert.

Filter im Sinne der Erfindung sind Filter, die in Absaugeinrichtungen, beispielsweise einer Arztpraxis und insbesondere einer zahnärztlichen Praxis zum Einsatz kommen. Zahnärztlich im Sinne dieses Patents sind auch kieferchirurgische oder kieferorthopädische Eingriffe. Die Filter dienen zum Herausfiltern von Feststoffpartikeln aus der Absauglösung, die mittels der Absaugeinrichtung aus dem Mund des Patienten abgesaugt wird. Die Flüssigkeit der Absauglösung besteht im Wesentlichen aus dem Speichel des Patienten sowie des Weiteren aus Spülflüssigkeit und gegebenenfalls Blut. Bei den Feststoffpartikeln, die herausgefiltert werden, kann es sich entweder um unerwünschte Partikel wie z.B. Amalgam handeln, die herausgefiltert werden, um sie entsorgen zu können, oder um solche, die erwünscht sind und die herausgefiltert werden, um sie einer weiteren Verwendung zuzuführen. Beispiele für gewünschte Partikel sind Knochenmaterial oder Edelmetalle. Der Filter der vorliegenden Erfindung ist insbesondere für das Herausfiltern von Edelmetallen geeignet, die beispielsweise in edelmetallhaltigen Dentallegierungen enthalten sind, wie sie in der EP 1 432 381 B1 beschrieben sind. Dabei ist vor allem Gold zu nennen. Aber auch für andere Edelmetalle, Knochenmaterial oder sonstige erwünschte Stoffe kann der Filter verwendet werden. Gewünschte Stoffe sind beispielsweise Stoffe, die man aus der Absaugflüssigkeit herausfiltern möchte, weil man sie weiterverwenden und/oder recyceln möchte. Darüber hinaus kann der Filter auch für Amalgam oder sonstige unerwünschte Partikel verwendet werden. Unerwünschte Partikel sind beispielsweise solche Partikel, die in der Absaugflüssigkeit enthalten sind, aber aus dieser herausgefiltert werden (müssen), bevor man die Absaugflüssigkeit dem Abwasser zuführen darf, die also aufgrund von Abwasserbestimmungen nicht in der Absaugflüssigkeit verbleiben dürfen, wenn diese dem Abwasser zugeführt werden soll.

Absaugeinrichtungen bestehen aus einem Vakuumerzeuger und einem Rohr-/ Schlauchsystem. Sie dienen dazu, bei Schleif- und Bohrarbeiten an den Zähnen eines Patienten entstehende Partikel aus dem Behandlungsbereich im Mundraum zu entfernen. Am einen Ende der Absaugeinrichtung befindet sich das Mundstück, das in den Mund des Patienten eingeführt wird. An das Mundstück schließt sich ein Schlauch an, auf den das Mundstück aufgesteckt ist. Am anderen Ende der Absaugeinrichtung ist üblicherweise ein Amalgamfilter angebracht, um Amalgamreste aus der Absauglösung herauszufiltern, bevor diese ins Abwasser entlassen wird. Im Amalgamfilter werden auch weitere Feststoffpartikel abgeschieden.

Der Filter der vorliegenden Erfindung besteht aus dem Filterkörper und dem Filtergewebe.

Der Filterkörper besteht vorzugsweise aus einem Kunststoffmaterial wie Polypropylen (PP), Polyethylen (PE), Polyetheretherketon (PEEK) oder Polyethylenterephthalat (PET), aber auch andere Materialien wie Metalle, zum Beispiel Gold, Titan, Stahl oder Edelstahl, oder beliebige Kombinationen aus zwei oder mehreren Materialien sind möglich. Das Material sollte vorzugsweise möglichst leicht sein, um die Handhabung der Absaugeinrichtung mit eingestecktem Filter zu erleichtern. Als Kunststoffmaterial ist insbesondere Polypropylen (PP) geeignet.

Der Filterkörper kann einteilig ausgebildet sein. Dann umfasst er das Filtergehäuse. Das Filtergehäuse ist als rohrförmiger Hohlkörper mit mindestens zwei, vorzugsweise genau zwei, Öffnungen ausgebildet, und zwar vorzugsweise in Strömungsrichtung gerade, so dass die beiden an den Enden befindlichen Öffnungen, die erste Gehäuseöffnung und die zweite Gehäuseöffnung, einander gegenüberliegen. Strömungsrichtung ist die Gesamtfließrichtung der Absauglösung vom Eingangsabschnitt zum Schlauchanschlussabschnitt, unabhängig von eventuell auftretenden einzelnen Verwirbelungen. Das Filtergehäuse kann jedoch auch gebogen oder in sonstiger Weise ausgeführt sein. Das Filtergehäuse kann einteilig oder mehrteilig ausgestaltet sein. Innen- und Außendurchmesser sowie die Wandstärke und die Geometrie des Querschnitts können über die Länge variieren. Der Querschnitt (senkrecht zur Strömungsrichtung) des Filtergehäuses kann verschiedene Geometrien aufweisen, vorzugsweise ist er kreisförmig ausgebildet. Das Filtergehäuse weist an der ersten Gehäuseöffnung einen Schlauchanschlussabschnitt und an der zweiten Gehäuseöffnung einen Eingangsabschnitt auf. An dem Schlauchanschlussabschnitt wird das Filtergehäuse mit dem einen Teil der Absaugeinrichtung verbunden, an dessen anderem Ende sich der Vakuumerzeuger befindet. Üblicherweise erfolgt die Verbindung mit der Absaugeinrichtung über einen Schlauch. An dem Eingangsabschnitt wird das Filtergehäuse mit dem anderen Ende des Teils der Absaugeinrichtung verbunden. Dabei handelt es sich um den Teil, an dessen anderem Ende sich das Mundstück befindet. Vorzugsweise ist der Eingangsabschnitt direkt mit dem Mundstück der Absaugeinrichtung verbunden und der Eingangsabschnitt ist so ausgebildet, dass er auf das Mundstück der Absaugeinrichtung gesteckt werden kann. Vorzugsweise ist der Eingangsabschnitt konisch, insbesondere sich nach zur zweiten Gehäuseöffnung hin erweiternd, ausgebildet. Durch eine solche Ausbildung passt der Eingangsabschnitt auf handelsübliche Mundstücke, ohne dass eine spezielle Anpassung erforderlich wäre. Ebenso ist der Schlauchanschlussabschnitt konisch, vorzugsweise sich zur ersten Gehäuseöffnung hin konisch verjüngend, ausgebildet. Auf diese Weise passt der Schlauchanschlussabschnitt auf handelsübliche Saugschläuche einer Absaugeinrichtung und auch hier ist eine spezielle Anpassung nicht erforderlich.

Am Eingangsabschnitt ist vorzugsweise auch das Filtergewebe angebracht, und zwar derart, dass die Öffnung des sackförmig ausgebildeten Filtergewebes in Strömungsrichtung vor dem geschlossenen Ende des sackförmig ausgebildeten Filtergewebes angeordnet ist. Vorteilhafter Weise bilden Eingangsabschnitt des Filtergehäuses und das Mundstück der Absaugeinrichtung die Befestigung des Filtergewebes. Die Verbindung von Eingangsabschnitt und Mundstück kann beispielsweise mittels Schraubverschluss, Bajonettverschluss oder magnetisch erfolgen. Besonders bevorzugt ist eine Klemmverbindung von Eingangsabschnitt und Mundstück. Zwischen den beiden Teilen wird dann das Filtergewebe eingeklemmt. Dies kann insbesondere dadurch erfolgen, dass das Mundstück an seinem dem Filter zugewandten Ende einen sich verjüngenden, vorzugsweise konisch verjüngenden, Abschnitt aufweist und die Innenwand des Eingangsabschnitts des Filtergehäuses dazu korrespondierend sich zur zweiten Gehäuseöffnung hin erweiternd ausgebildet ist. Alternativ kann ist die Innenwand des besagten Abschnitts des Mundstücks sich zu seinem dem Filtern zugewandten Ende hin erweiternd, vorzugswürdig konisch erweiternd, und die Außenwand des Eingangsabschnitts des Filtergehäuses dazu korrespondierend sich zur zweiten Gehäuseöffnung hin verjüngend ausgebildet sein.

In einer besonders bevorzugten Ausführungsform ist der Filterkörper zweiteilig ausgebildet und umfasst das Filtergehäuse sowie eine als Hohlkörper mit mindestens zwei Öffnungen ausgebildete Filterhülse mit einem Mundstückanschlussabschnitt an einer ersten Hülsenöffnung und mit einem Gehäuseverbindungsabschnitt, der zur Verbindung mit dem Eingangsabschnitt des Gehäuses ausgebildet ist, an einer zweiten Hülsenöffnung.

Die Filterhülse ist als rohrförmiger Hohlkörper mit mindestens zwei, vorzugsweise genau zwei, Öffnungen, einer ersten und einer zweiten Hülsenöffnung, ausgebildet, und zwar vorzugsweise in Strömungsrichtung gerade, so dass die beiden an den Enden befindlichen Hülsenöffnungen einander gegenüberliegen. Die Filterhülse kann jedoch auch gebogen oder in sonstiger Weise ausgeführt sein. Die Filterhülse kann einteilig oder mehrteilig ausgebildet sein. Innen- und Außendurchmesser sowie die Wandstärke der Filterhülse können über die Länge variieren. Der Querschnitt (senkrecht zur Strömungsrichtung) der Filterhülse kann verschiedene Geometrien aufweisen, vorzugsweise ist er kreisförmig ausgebildet. Die Geometrie des Querschnitts kann über die Länge variieren. Die Filterhülse weist an der ersten Hülsenöffnung einen Mundstückanschlussabschnitt und an der zweiten Hülsenöffnung einen Gehäuseverbindungsabschnitt auf. An dem Mundstückanschlussabschnitt werden die Filterhülse und damit der Filterkörper mit dem einen Ende des Teil der Absaugeinrichtung verbunden, an dessen anderem Ende sich das Mundstück befindet. Vorzugsweise ist der Mundstückanschlussabschnitt direkt mit dem Mundstück der Absaugeinrichtung verbunden und der Mundstückanschlussabschnitt ist so ausgebildet, dass er auf das Mundstück der Absaugeinrichtung gesteckt werden kann. Vorzugsweise ist der Mundstückanschlussabschnitt konisch, insbesondere sich zur ersten Hülsenöffnung hin konisch erweiternd, ausgebildet. Durch eine solche Ausbildung passt der Mundstückanschlussabschnitt auf handelsübliche Mundstücke, ohne dass eine spezielle Anpassung erforderlich wäre.

Vorteilhafter Weise bilden Eingangsabschnitt des Filtergehäuses und der Gehäuseverbindungsabschnitt der Filterhülse im montierten Zustand die Befestigung des Filtergewebes. Die Verbindung von Eingangsabschnitt und Gehäuseverbindungsabschnitt kann beispielsweise mittels Schraubverschluss, Bajonettverschluss oder magnetisch erfolgen. Durch die Verbindung wird das Filtergewebe zwischen den beiden Teilen fixiert. Besonders bevorzugt ist eine Klemmverbindung von Eingangsabschnitt und Gehäuseverbindungsabschnitt.

Zwischen den beiden Teilen wird dann das Filtergewebe eingeklemmt. Im diesem Fall kann der Eingangsabschnitt des Filtergehäuses auch als "Filterklemmbereich" des Filtergehäuses bezeichnet werden. Entsprechend kann der Gehäuseverbindungsabschnitt der Filterhülse als "Filterklemmbereich" der Filterhülse bezeichnet werden. Auch beliebige Kombinationen der Verschlussmöglichkeiten sind denkbar.

Vorteilhafterweise sind diejenigen Abschnitte von Filtergehäuse und/oder Filterhülse, die mit den zugehörigen Abschnitten der Absaugeinrichtung verbunden werden, so ausgebildet, dass ein direkter Anschluss an die Absaugeinrichtung möglich ist. Ebenso ist es jedoch möglich, dass ein Adapterstück zwischen Abschnitt am Filter und korrespondierendem Abschnitt an der Absaugeinrichtung vorgesehen ist.

Vorzugsweise ist die Außenwand des Gehäuseverbindungsabschnittes der Filterhülse sich zur zweiten Hülsenöffnung hin verjüngend, besonders bevorzugt konisch verjüngend, und die Innenwand des Eingangsabschnitts des Filtergehäuses dazu korrespondierend sich zur zweiten Gehäuseöffnung hin erweiternd, besonders bevorzugt konisch erweiternd, ausgebildet. Alternativ kann die Innenwand des Gehäuseverbindungsabschnittes der Filterhülse sich zur zweiten Hülsenöffnung hin erweiternd, vorzugswürdig konisch erweiternd, und die Außenwand des Eingangsabschnitts des Filtergehäuses dazu korrespondierend sich zur zweiten Gehäuseöffnung hin verjüngend ausgebildet sein.

In einer bevorzugten Ausführungsform ist am Gehäuseverbindungsabschnitt der Filterhülse eine Filtersackanbringhilfe vorgesehen. Diese ermöglicht es, das sackförmige Filtergewebe einfacher an dem Gehäuseverbindungsabschnitt der Filterhülse anzubringen. Für den Fall, dass der Gehäuseverbindungsabschnitt sich zur zweiten Hülsenöffnung konisch verjüngend ausgebildet ist, kann die Filtersackanbringhilfe zum Beispiel in Form von sich von dem Gehäuseverbindungsabschnitt in Längsrichtung erstreckenden, am Ende nach innen zulaufenden Dornen gebildet sein. Diese erleichtern ein Öffnen des Filtersacks und ein Aufschieben des Filtersacks auf den Gehäuseverbindungsabschnitt. Weiterhin unterstützen sie, dass der Filtersack offen gehalten wird. Die Dornen sind vorzugsweise flexibel ausgebildet und können in Länge und Stärke variieren. Wenn die Filtersackanbringhilfe in Form von Dornen ausgebildet ist, sollten mindestens zwei Dornen vorgesehen sein, wobei mindestens drei Dornen besonders gut geeignet sind. Die Länge der Dornen ist nach oben durch die Länge des Filtergewebesacks begrenzt. Im Hinblick auf die Mindestlänge sind solche Ausführungen besonders bevorzugt, bei denen die Dornenlänge mindestens ein Viertel, insbesondere mindestens ein Drittel, der Länge des Filtergewebesacks betragen.

Besonders bevorzugt weist das Filtergehäuse einen Umströmungsbereich auf, der in Strömungsrichtung hinter dem Eingangsabschnitt oder Filterklemmbereich liegt und sich an diesen anschließt, wobei der Umströmungsbereich einen größeren Innendurchmesser aufweist als der Eingangsabschnitt und wobei der Übergang zwischen Eingangsabschnitt und Umströmungsbereich unter Bildung eines Absatzes erfolgt. Das heißt, dass der Übergang zwischen Eingangsabschnitt und Umströmungsbereich nicht stetig ausgebildet ist. Der Absatz kann auch abgerundet sein. Durch den Absatz ist gewährleistet, dass der Filtersack im Umströmungsbereich nicht an der Filtergehäusewand anliegt, was eine Verringerung der offenen Filterfläche mit sich brächte und daher nachteilig wäre. Vorzugsweise erfolgt eine Vergrößerung des Innendurchmessers vom Ende des Eingangsabschnitts zum Beginn des Umströmungsbereiches auf das 1,3- bis 2,0-fache, insbesondere auf das 1,4 bis 1,7-fache, besonders bevorzugt auf das 1,5- bis 1,6-fache und ganz besonders bevorzugt auf das 1,55-fache.

An seinem anderen Ende schließt sich an den Umströmungsbereich der Schlauchanschlussabschnitt an, der somit in Strömungsrichtung hinter dem Umströmungsbereich liegt und den Umströmungsbereich an diesem Ende begrenzt. Vorzugsweise erfolgt eine Verringerung des Innendurchmessers vom Ende des Umströmungsbereiches zum Beginn des Schlauchanschlussabschnittes um das 1,5- bis 2,5-fache, insbesondere um das 1,7 bis 2,3-fache, besonders bevorzugt um das 1,8- bis 2,3-fache.

Für das Strömungsverhalten der Absaugflüssigkeit ist es besonders günstig, wenn die Filtergehäusewand im Anschluss an den Absatz nicht gerade verläuft, sondern sich konisch erweitert. Diese konische Erweiterung bewirkt, dass weniger Strömungswirbel entstehen, und vergrößert die Strömungsgeschwindigkeit kurz hinter dem Übergang von Eingangsabschnitt zu Umströmungsbereich. Auf diese Weise wird die Absaugflüssigkeit zügiger durch den Filter gezogen. Die Saugleistung des Filters wird also besonders stark erhöht.

Die beschriebene Ausgestaltung führt darüber hinaus dazu, dass sich leichtere Partikel im oberen Bereich des Filtersackes ablagern, während schwerere Partikel bis in den unteren (Boden)Bereich des Filtergewebesacks gelangen. Mit "oberem Bereich" ist dabei der Bereich um die Öffnung des Filtersackes gemeint, mit "unterem (Boden)Bereich" der der Öffnung gegenüberliegende Bereich.

Der Umströmungsbereich kann darüber hinaus zwei Abschnitte aufweisen, wobei der Übergang vom ersten Umströmungsbereichsabschnitt zum zweiten Umströmungsbereichsabschnitt, der in Strömungsrichtung hinter dem ersten Umströmungsbereichsabschnitt angeordnet ist, ebenfalls unter Bildung eines Absatzes erfolgt und der zweite Umströmungsbereichsabschnitt vorteilhafter Weise einen größeren Innendurchmesser aufweist als der erste Umströmungsbereichsabschnitt. Eine solche Ausbildung ist besonders günstig, da sie nochmals verbesserte Strömungseigenschaften mit sich bringt. Auch für den zweiten Umströmungsbereichsabschnitt ist eine konische Erweiterung der Filtergehäusewand hinter dem gebildeten Absatz für die Strömungseigenschaften besonders vorteilhaft.

Das Filtergehäuse kann verschiedene Geometrien aufweisen. So ist beispielsweise ein quadratischer oder ein ovaler Querschnitt möglich. Besonders bevorzugt ist eine rotationssymmetrische Ausbildung des Filtergehäuses, also die Ausbildung des Filtergehäuses mit einem kreisförmigen Querschnitt. Dies macht die Handhabung besonders angenehm und erweitert darüber hinaus die Palette der möglichen Herstellungsverfahren. Der Durchmesser des Querschnitts kann über die Länge des Gehäuses variieren. Auch die Filterhülse kann rotationssymmetrisch ausgebildet sein. Sowohl der Querschnitt des Filtergehäuses wie auch derjenige der Filterhülle können über die Länge variieren.

In einer bevorzugten Ausführungsform folgt die äußere Form des Filtergehäuses der inneren Form. In Strömungsrichtung hinter dem Eingangsabschnitt erweitert sich der Außendurchmesser des Filtergehäuses unter Bildung eines Absatzes. Es schließt sich der Umströmungsbereich an, dessen Außendurchmesser über die Länge in Strömungsrichtung geringfügig zunimmt. Vorzugsweise ist der Außendurchmesser des Umströmungsbereiches im Bereich seines größten Umfangs um den Faktor 1,3 bis 2,0, vorzugsweise um den Faktor 1,4 bis 1,7, besonders bevorzugt um den Faktor 1,5 bis 1,6 größer als der Außendurchmesser des Schlauchanschlussabschnittes. Am Ende des Umströmungsbereiches befindet sich auch an der Außenseite ein weiterer Absatz zum sich anschließenden Schlauchanschlussabschnitt, der einen geringeren Außendurchmesser aufweist als der Umströmungsbereich. Vorzugsweise ist der Außendurchmesser des Umströmungsbereiches im Bereich seines größten Umfanges um den Faktor 1,5 bis 2,5, vorzugsweise um den Faktor 1,7 bis 2,3, besonders bevorzugt um den Faktor 1,9 bis 2,1 größer als der Außendurchmesser des Schlauchanschlussabschnittes. Der "Bereich des größten Umfanges" bezeichnet dabei den Bereich des Filtergehäuses, an dem der Außendurchmesser am größten ist, wobei etwaige aus der Gehäusewand hervorstehende Vorsprünge nicht berücksichtigt sind.

Eine Länge des Filtergehäuses (gemessen vom äußeren Ende des Eingangsabschnitts bis zum äußeren Ende des Schlauchanschlussabschnitts) im Bereich von 5 bis 15 cm, insbesondere von 6 bis 12 cm und besonders bevorzugt von 7 bis 10 cm ermöglicht eine besonders ergonomische Ausbildung des Filters. Vorzugsweise beträgt die Länge des Umströmungsbereiches 3 bis 9 cm, insbesondere 3,5 bis 7 cm und insbesondere 4 bis 5 cm. Der Filter bildet auf diese Weise einen Bereich, an dem sich die Absaugeinrichtung sehr gut handhaben lässt. Im Falle einer zahnärztlichen Absaugeinrichtung ist die Handhabung auch unabhängig davon, wie der Benutzer gewohnt ist, das Mundstück einer Absaugeinrichtung zu führen, sei es von oben gegriffen zwischen Daumen und Zeigefinger, von unten gegriffen zwischen Daumen und Zeigefinger oder von unten gegriffen analog zur Schreibhaltung eines Stiftes. Der zwischen Mundstück und Schlauch eingefügte Filter verändert die gewohnte Handhabung nicht.

In einer weiteren bevorzugten Ausführungsform erstreckt sich der eingesetzte Filtersack im ausgestreckten Zustand bei durchfließender Strömung über zwischen 70% und 95%, vorzugsweise über zwischen 75% und 90% und insbesondere zwischen 80% und 95% der Länge des Umströmungsbereichs. Der Filtersack erstreckt sich also nicht über die gesamte Länge des Umströmungsbereiches, sondern endet in einem Abstand von dem Schlauchanschlussabschnitt. Diese Ausbildung unterstützt die Strömung zusätzlich, weil die Luft relativ ungehindert am Sack entlanggleiten kann.

Der Begriff "Filtergewebe" im Sinne der Erfindung umfasst sowohl Gewebe wie auch Gewirke oder eine Einheit von auf anderem Wege miteinander verbundenen Einzelfäden, wobei Gewebe und Gewirke und insbesondere Gewebe besonders bevorzugt sind.

Das Filtergewebe ist ein feinmaschiges Gewebe, das vorzugsweise aus einem Kunststoffmaterial besteht. Dabei sind Polyethlenterephthalat (PET) und insbesondere Polyamid (PA) besonders bevorzugt. Das Filtergewebe kann aber auch aus anderen Materialien wie z.B. Glasfasern, Stofffasern, Metalldraht, insbesondere aus Stahl, Edelstahl, Federstahl, Kupfer, Messing, verchromtem Messing, Zinn-Bronze, Nickel, Monel oder Aluminium, oder anderen Kunststoffen wie insbesondere Polypropylen (PP), Polystyrol (PS), Polycarbonat (PC), Ethylen-Chlortrifluorethylen-Fluorcopolymer (E-CTFE), Ethylen-Tetrafluorethylen (ETFE) oder Polyetheretherketon (PEEK), oder aus Materialkombinationen aus zwei oder mehr der genannten Materialien bestehen. Bei den Metalldrähten sind verchromtes Messing, Aluminium und Edelstahl bevorzugt.

Besonders bevorzugt besteht das Filtergewebe aus einem Material, das beim Einschmelzen der aufgefangenen Partikel, insbesondere des aufgefangenen Edelmetalls bzw. der aufgefangenen Dentallegierung rückstandslos verbrennt. Vorzugsweise weist das Filtergewebematerial eine Zersetzungstemperatur, d.h. eine Temperatur, oberhalb derer in Gegenwart von Luft ein Zerfall der Makromoleküle in kleinere Einheiten eintritt, oder eine Zündtemperatur, d.h. eine Temperatur, auf die man einen Stoff erhitzen muss, damit sich eine brennbare Substanz in Gegenwart von Luft ausschließlich aufgrund ihrer Temperatur - also ohne Zündquelle wie einen Zündfunken - selbst entzündet, auf, die unterhalb der Schmelztemperatur der aufgefangenen Partikel liegt, da dann bei Einschmelzen der aufgefangenen Partikel das Filtergewebe in jedem Fall verbrennt. Insbesondere edelmetallhaltige Dentallegierungen gehören zu den aufgefangenen Partikeln. Die Schmelzintervalle edelmetallhaltiger Dentallegierungen liegen üblicherweise zwischen 900°C und 1.800°C. Der Schmelzpunkt von Gold beträgt 1.063°C. Die Zersetzungstemperatur der meisten Kunststoffe liegt zwischen 200°C und 600 °C, so dass Kunststoffe als Filtergewebematerial besonders geeignet sind.

Im Falle der Verwendung von Metalldrähten als Filtergewebematerial ist es besonders bevorzugt, wenn sich der Schmelzpunkt des Metalldrahtes von demjenigen der einzuschmelzenden edelmetallhaltigen Dentallegierung hinreichend unterscheidet, so dass entweder die Dentallegierung deutlich vor dem Metalldraht schmilzt oder umgekehrt der Metalldraht deutlich vor der Dentallegierung schmilzt, um eine einfache Trennung der Dentallegierung von dem Filtergewebematerial zu ermöglichen. Vorzugsweise unterscheiden sich die Schmelzpunkte um mindestens 100°C, besonders bevorzugt um mindestens 200 °C. Eine weitere Möglichkeit bei der Verwendung von Metalldrähten besteht darin, ein Material für das Filtergewebe zu verwenden, das in üblichen Dentallegierungen enthalten ist wie z.B. Kupfer, Zinn oder Zink. Das Filtergewebematerial würde dann mit den aufgefangenen Partikeln zusammen eingeschmolzen und recycelt.

Das Filtergewebe kann als Einmalfiltergewebe oder als Mehrfachfiltergewebe ausgebildet sein. Im ersten Fall verbleiben die entnommenen Partikel im Filtergewebesack und werden in einem Sammelbehälter aufbewahrt. Im letzteren Fall wird der Filtergewebesack nach der Benutzung entleert, und nur die entnommenen Partikel werden in einem Sammelbehälter aufbewahrt, während der Filtergewebesack desinfiziert oder sterilisiert wird.

In beiden Fällen wird das entnommene Material einem Recyclingverfahren zugeführt.

Eine Verwendung eines Einmalfiltergewebes, das verbrannt werden kann, ist für das Recycling besonders günstig, da gewährleistet ist, dass das gesamte im Filter aufgefangene Material recycelt wird und keine Verluste z.B. durch Rückstände im Filter bei dessen Entleeren, eintreten.

Ein Filtergewebe ist durch bestimmte Parameter definiert wie insbesondere das Material, die Maschenweite w (üblicherweise angegeben in $\mu$m), den Fadendurchmesser d (üblicherweise angegeben in $\mu$m) und die offene Fläche $a_o$ (üblicherweise angegeben in %). Diese Begriffe entsprechen den Begrifflichkeiten nach DIN 4185-1 und DIN ISO 9044.

Demnach ist die Maschenweite w der Abstand zwischen zwei benachbarten Kett- oder Schussfäden, in der Projektionsebene und etwa mittig der Masche gemessen. Kettfäden sind parallel zur Webrichtung angeordnet und Schussfäden rechtwinklig zur Webrichtung. Zur Feststellung der Maschenweite sind verschiedene Messmethoden möglich. So kann beispielsweise zur Feststellung der Maschenweite in Kettrichtung zunächst die Länge eine Messstrecke über mehrere (vorzugswürdig mindestens 20) benachbarte Maschen und Fäden in Kettrichtung gemessen werden. Die festgestellte Länge wird durch die Anzahl der Maschen und Fäden (im vorgenannten Beispiel 20) geteilt. Um die Maschenweite zu erhalten, wird von diesem Ergebnis der zuvor festgestellte Fadendurchmesser abgezogen.

Der Fadendurchmesser nach dem Verweben kann beispielsweise mittels einer Mikrometerschraube oder einem Messschieber ermittelt werden.

Die "offenen Fläche" $a_o$ eines Filtergewebes ist die Gesamtfläche des Gewebes abzüglich derjenigen Fläche, die vom Faden des Filtergewebes eingenommen wird. Die Angabe der offenen Fläche erfolgt in Prozent. Die offene Fläche ist abhängig vom Fadendurchmesser d des im Filtergewebe verwendeten Fadens und von der Maschenweite w des Filtergewebes. Die offene Fläche berechnet sich wie folgt:

$$(a_o)[\%] = \frac{(w)^2 \times 100}{(w + d)^2}$$

In dem Filtergewebe werden die abzutrennenden Partikel aufgefangen. Ist die gewünschte Menge an Partikeln im Filtergewebe aufgefangen, wird das Filtergewebe ausgewechselt. In einer bevorzugten Ausführungsform ist der Filterkörper ganz oder teilweise durchscheinend, so dass ohne Öffnen des Filterkörpers der Füllstand des Filtergewebes sichtbar ist oder mit einer Anzeige versehen, die unabhängig von der Saugleistung Aussagen über den Füllstand oder sonstige Parameter trifft und darauf aufmerksam macht, dass das Filtergewebe voll ist und ausgewechselt werden muss. Es können also weitere Anzeigen, sei es optisch oder akustisch, vorgesehen sein, die neben dem Umstand, dass die Saugleistung nachlässt, darauf hinweisen, dass das Filtergewebe ausgewechselt werden sollte.

Vorzugsweise sind Fadendurchmesser und Maschenweite über das gesamte Gewebe im Wesentlichen einheitlich, besonders vorzugswürdig identisch. Vorzugswürdig besteht das Filtergewebe aus Quadratmaschen, es sind aber auch andere Maschenformen wie z.B. Langmaschen, Breitmaschen oder Nullmaschen sowie Kombinationen verschiedener Maschenformen möglich. Das Filtergewebe kann auch aus zwei oder mehreren übereinander liegenden gleichen oder unterschiedlichen Filtergeweben bestehen. Bei zwei oder mehreren übereinanderliegenden Filtergeweben ist es erfindungsgemäß, wenn nur eines der Filtergewebe die erfindungsgemäße Maschenweite aufweist oder wenn die erfindungsgemäße Maschenweite in der Projektion von zwei oder mehreren Filtergeweben zusammen auftritt.

Das erfindungsgemäße Filtergewebe ist sackförmig ausgebildet. Sackförmig bedeutet dabei eine Ausbildung in Form eines Sackes, eines Beutels oder einer Tasche, d.h. eines verformbaren oder formsteifen Behältnisses, das nur eine Öffnung aufweist. Der Filtergewebesack weist vorzugsweise möglichst wenige Nähte auf. Sofern Nähte vorhanden sind, sind diese bevorzugt überlappend und nicht aneinanderstoßend ausgebildet. Eine Ausbildung ohne jegliche Naht wäre unter technischen Gesichtspunkten besonders bevorzugt, ist jedoch aus Kostengründen zumeist nicht zweckmäßig. Daher werden üblicherweise Säcke mit einer Naht oder mit zwei Nähten zum Einsatz kommen. Im Falle nur einer Naht wird diese sich üblicherweise an der der Öffnung des Sackes gegenüberliegenden Seite befinden, also als Bodennaht ausgebildet sein, da zur Herstellung ein schlauchförmiges Gewebe verwendet wird, das an einem Ende durch eine Naht geschlossen wird. Im Falle von zwei Nähten wird zumeist ein flaches Gewebestück zunächst zu einem Schlauch geschlossen. Im Anschluss wird eine der beiden Schlauchöffnungen durch eine weitere Naht verschlossen. Für die Filterleistung ist es vorzuziehen, die Nahtlänge so gering wie möglich zu halten. Anstelle einer überlappenden Nahtbildung ist es auch möglich, die Gewebestücke mittels Laserschnitt miteinander zu verbinden. Dabei werden die Stücke gleichzeitig geschnitten und miteinander verbunden. Es sind auch Ausführungsformen möglich, bei denen einzelne Gewebestücke in anderer Form miteinander zu einem Filtergewebe zusammengefügt werden. Die einzelnen Gewebestücke müssen nicht durch eine Naht miteinander verbunden werden und müssen sich auch nicht berühren. Die Gewebestücke können auch in anderer Art und Weise, z.B. über Kunststoffschienen, miteinander verbunden werden.

Das Filtergewebe kann einlagig und im Wesentlichen glatt ausgebildet sein. Ebenso ist es möglich, das Filtergewebe zumindest teilweise plissiert, also in Falten gelegt, einzusetzen.

Erfindungsgemäß beträgt die offene Fläche des Filtergewebes mindestens das Doppelte der Fläche des Querschnitts des äußeren Endes des Schlauchanschlussabschnitts, vorzugsweise beträgt sie das 2,5-fache und besonders bevorzugt das Dreifache der Fläche des Querschnitts des äußeren Endes des Schlauchanschlussabschnitts.

Erfindungsgemäß beträgt die Maschenweite w des Filtergewebes in Kett- (parallel zur Webrichtung) und in Schussrichtung (rechtwinklig zur Webrichtung) jeweils 10-80 $\mu$m, vorzugsweise beträgt sie 15-60 $\mu$m und besonders bevorzugt 19-30 $\mu$m. Es ist unschädlich, wenn einzelne Maschenweiten außerhalb des genannten Bereichs liegen.

Der Fadendurchmesser d der Fäden des Filtergewebes beträgt erfindungsgemäß 10-80 $\mu$m, vorzugsweise 15-60 $\mu$m und besonders bevorzugt 19-40 $\mu$m. Der Fadendurchmesser bezieht sich in der Regel auf die Fäden im unverwebten Zustand. Es ist aber ebenso erfindungsgemäß, wenn die Fäden im verwebten und/oder benutzten Zustand den beschriebenen Fadendurchmesser aufweisen. Es ist unschädlich, wenn an einzelnen Stellen der Fadendurchmesser außerhalb des genannten Bereichs liegt. Bei nicht kreisrunden Fadenquerschnitten ist es erfindungsgemäß, wenn der Durchmesser eines gedachten Kreises, der durch die zwei äußersten Punkte gebildet wird, in den beschriebenen Bereich fällt.

Kett- und Schussfäden können denselben Fadendurchmesser aufweisen. Ebenso ist es möglich, dass sich der Durchmesser des Kettfadens von demjenigen des Schussfadens unterscheidet. Besonders bevorzugt ist es, wenn bei unterschiedlichem Fadendurchmesser von Kett- und Schussfaden der Durchmesser des Kettfadens 20-80 $\mu$m, vorzugsweise 20-40 $\mu$m und besonders bevorzugt 20-30 $\mu$m und der des Schussfadens weniger als 30 $\mu$m, vorzugsweise weniger als 20 $\mu$m und besonders bevorzugt zwischen 15 und 19 $\mu$m beträgt. Eine solche Ausführungsform ermöglicht ein stabiles engmaschiges Gewebe mit gleichzeitig verhältnismäßig großer offener Fläche.

Mit diesen Parametern wird eine gute Saugleistung gewährt, während gleichzeitig bis zu 90% oder mehr des abgesaugten Edelmetallstaubs aufgefangen werden. Dies stellt eine große Steigerung im Vergleich zu den Filtern des Standes der Technik dar, die nur ca. 50% des abgesaugten Edelmetallstaubs auffangen können.

Bei der Verwendung des Filters im ärztlichen/zahnärztlichen Bereich kann es gewünscht sein, den Filter nach seiner Verwendung zu desinfizieren, bevor er erneut eingesetzt wird. Dazu ist es möglich, den Filter in eine Desinfektionsflüssigkeit zu legen. Vorzugsweise ist das Filtermaterial autoklavenbeständig, d.h. es ist beständig gegenüber einer Wärmebehandlung unter Druck. Durch das Autoklavieren bei einer Temperatur von mindestens 134°C kann der Filter dann sterilisiert werden.

Auch das Filtergewebe ist vorzugsweise autoklavenbeständig. Für den Fall, dass es sich bei dem Filtergewebe um ein Einmalprodukt handelt, ist ein Sterilisieren des Filtergewebes zwar nicht zwangsläufig erforderlich. Jedoch ist angesichts des Umstandes, dass das Filtergewebe mit den aufgefangenen gewünschten Partikeln einem Sammelbehälter zugeführt wird, aus hygienischen Gründen eine Sterilisation wünschenswert.

Die Erfindung betrifft darüber hinaus eine zahnärztliche Absaugeinrichtung, umfassend einen Vakuumerzeuger, ein Rohr-/Schlauchsystem und ein Mundstück, die darüber hinaus einen erfindungsgemäßen Filter umfasst. Vorzugsweise arbeitet die Absaugeinrichtung mit einem Unterdruck des Vakuumerzeugers von 70 bis 200 mbar. Der gewünschte Unterdruck kann am Vakuumerzeuger frei eingestellt werden. Der Unterdruck bestimmt die Saugleistung der Absaugeinrichtung. Allerdings hängt die Saugleistung von weiteren Parametern ab wie der Dichtigkeit des gesamten Systems einschließlich der Schläuche und Rohre sowie etwaiger Verbindungsstücke. Wie oben ausgeführt, hat der Filter einen maßgeblichen Einfluss auf die Saugleistung. Je kleiner die offene Fläche des im Filter enthaltenen Filtergewebes ist, desto schwächer ist die Saugleistung der Absaugeinrichtung. Im Betrieb verringert sich die offene Fläche des Filtergewebes durch die vom Filtermaterial herausgefilterten Partikel.

Des Weiteren betrifft die vorliegende Erfindung eine Filterhülse als Einführhilfe für einen Filtersack in einen Filter einer Absaugeinrichtung, insbesondere zur Verwendung in einem Filter wie vorstehend beschrieben, wobei die Filterhülse als Hohlkörper mit mindestens zwei Öffnungen ausgebildet ist, wobei sich an einer ersten der mindestens zwei Öffnungen ein Mundstückanschlussabschnitt zur Verbindung der Hülse mit einer Seite einer Absaugeinrichtung befindet und sich an einer zweiten der mindestens zwei Öffnungen ein Filteranbringabschnitt befindet, an dem der Filtersack an der Filterhülse angebracht wird. Eine solche Filterhülse weist erfindungsgemäß am Filteranbringabschnitt eine Filtersackanbringhilfe auf, wobei der Filteranbringabschnitt zur Verbindung mit dem Filter ausgebildet ist. Die Filterhülse und ihre weiteren vorteilhaften Ausgestaltungen sind weiter oben im Zusammenhang mit dem Filter bereits beschrieben. Auf diese Beschreibung wird zur Vermeidung von Wiederholungen verwiesen. Dabei entspricht der Filteranbringabschnitt dem Gehäuseverbindungsabschnitt.

Bei der Verwendung des erfindungsgemäßen Filters in einer Absaugvorrichtung kann es erforderlich sein, den Filter im Betrieb zu wechseln. Wird der Filter zum Beispiel in einer zahnärztlichen Absaugeinrichtung zum Absaugen von Goldpartikeln eingesetzt, kann es sein, dass die Menge an abzusaugendem Gold so groß ist, dass die Saugleistung der Absaugeinrichtung für den Benutzer nicht mehr ausreichend ist und er deshalb zum Absaugen der weiteren Goldpartikel den Filter wechseln möchte. Dazu besteht entweder die Möglichkeit, den gesamten Filter auszuwechseln. Ebenso ist es möglich, das Filtergehäuse am Absaugschlauch zu belassen und lediglich die Filterhülse mit dem Filtersack auszuwechseln. Hülse und Sack werden aus dem Gehäuse gezogen und ein neuer Sack, der auf einer weiteren Hülse aufgezogen ist, wird in das Gehäuse gesteckt.

Schließlich betrifft die vorliegende Erfindung ein Verfahren zum Gewinnen und Wiederverwerten von Edelmetallen, insbesondere von Gold oder goldhaltigen Legierungen, aus zahnärztlichen Absauglösungen, insbesondere unter Verwendung eines vorstehend beschriebenen Filters. Das Verfahren umfasst die folgenden Schritte:

a) Aufziehen eines Filtergewebesackes auf eine Filterhülse auf einen Gehäuseverbindungsabschnitt;

b) Einführen des Gehäuseverbindungsabschnitts der Filterhülse in einen Hülsenverbindungsabschnitt eines Filtergehäuses unter Zusammendrücken des Filtergewebesacks und Zusammenfügen von Filterhülse und Filtergehäuse zum Filter unter klemmender Befestigung des Filtergewebesacks;

c) Einfügen des zusammengesetzten Filters in eine zahnärztliche Absaugeinrichtung zwischen Mundstück und Schlauch;

d) Absaugen von Edelmetallpartikel enthaltender Absaugflüssigkeit;

e) Auffangen der Edelmetallpartikel in dem Filtergewebesack;

f) Herausnehmen des Filters aus der Absaugeinrichtung zwischen Mundstück und Schlauch.

Das Aufziehen des Filtergewebesackes auf den Gehäuseverbindungsabschnitt in Schritt a) kann erleichtert werden, indem sich an dem Gehäuseverbindungsabschnitt eine Filtersackanbringhilfe befindet, die zum Beispiel in Form von sich von dem Gehäuseverbindungsabschnitt in Strömungsrichtung erstreckenden nach Innen zulaufenden Dornen gebildet sein kann. Weitere Ausgestaltungen sind vorstehend beschrieben.

Dabei ist es in Schritt f) möglich, den gesamten Filter herauszunehmen. Ebenso besteht die Möglichkeit, nur die Filterhülse mit dem Filtergewebesack zu entnehmen und gegen eine weitere Filterhülse mit einem frischen Filtergewebesack auszutauschen. Dies ist insbesondere dann zweckmäßig, wenn weitere Edelmetallpartikel abzusaugen sind, der zunächst verwendete Filtergewebesack aber beispielsweise schon gefüllt ist.

In einer bevorzugten Ausführungsform umfasst das Verfahren ferner die folgenden Schritte:

g) Sterilisieren mindestens eines der Teile Filtergehäuse, Filterhülse und Filtergewebesack;
h) Zuführen des Edelmetallpartikel enthaltenden Filtergewebesacks zu einem Sammelbehälter.

Der Filter kann in zusammengebautem Zustand der Absaugeinrichtung entnommen werden und in diesem Zustand sterilisiert werden. Es ist auch möglich, die einzelnen Teile des Filters, Filtergehäuse, Filterhülse und Filtergewebesack mit Edelmetallpartikeln getrennt zur sterilisieren. Das Sterilisieren kann insbesondere durch Autoklavieren erfolgen.

Besonders bevorzugt umfasst das Verfahren darüber hinaus die folgenden Schritte:

i) Transport des Sammelbehälters nach Erreichen einer gewünschten gesammelten Menge an Edelmetallpartikel enthaltenden Filtergewebesäcken zu einer Recyclingeinrichtung;
j) Wiedergewinnen von Edelmetall aus den Edelmetallpartikeln durch Aufschmelzen in der Recyclingeinrichtung.

Die Recyclingeinrichtung kann sich in unmittelbarer Nähe der Absaugeinrichtung befinden. Ebenso ist es möglich, dass Recyclingeinrichtung und Absaugeinrichtung räumlich getrennt sind.

Die im Folgenden anhand der beigefügten Zeichnung beschrieben weiteren Merkmale oder Weiterbildungen der Erfindung stellen jeweils für sich betrachtet oder in Kombination miteinander bevorzugte Ausführungsformen der Erfindung dar.

Es zeigen

Fig. 1 eine Schnittansicht einer Ausführungsform des erfindungsgemäßen Filtergehäuses;

Fig. 2 eine Schnittansicht einer Ausführungsform der erfindungsgemäßen Filterhülse;

Fig. 3 die in Fig. 2 gezeigte Filterhülse mit aufgestecktem Filtersack;

Fig. 4 eine Schnittansicht der in den Fig. 1-3 gezeigten Ausführungsform des erfindungsgemäßen Filters mit Filtersack in montiertem Zustand;

Fig. 5 die in Fig. 4 gezeigte Ausführungsform in Explosionsdarstellung;

Fig. 6 einen Teil einer erfindungsgemäßen Absaugeinrichtung mit zwischengestecktem erfindungsgemäßen Filter;

Fig. 7 eine erfindungsgemäße Absaugeinrichtung mit zwischengestecktem erfindungsgemäßen Filter einschließlich der Einheit mit Vakuumpumpe.

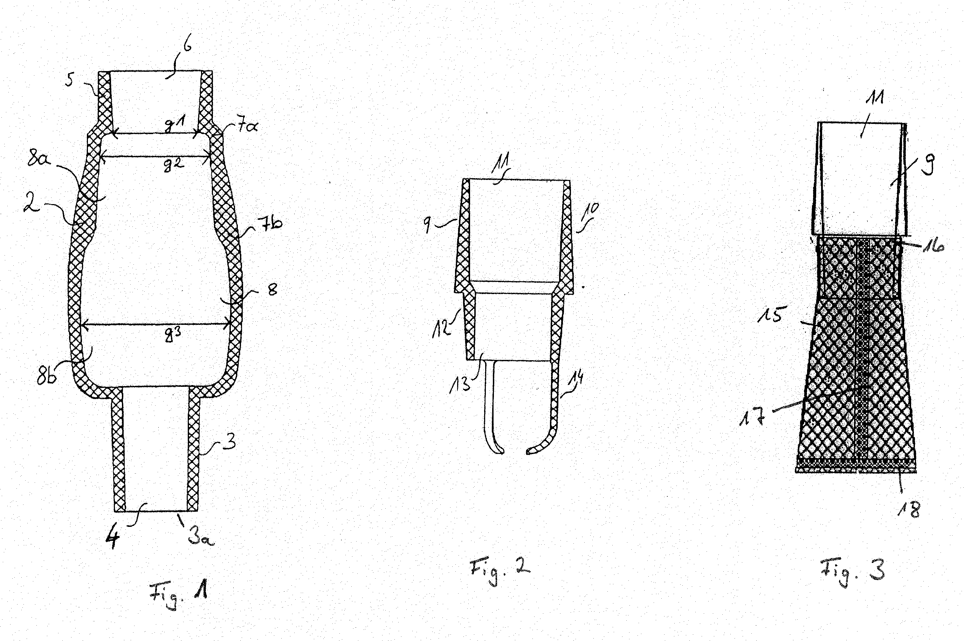

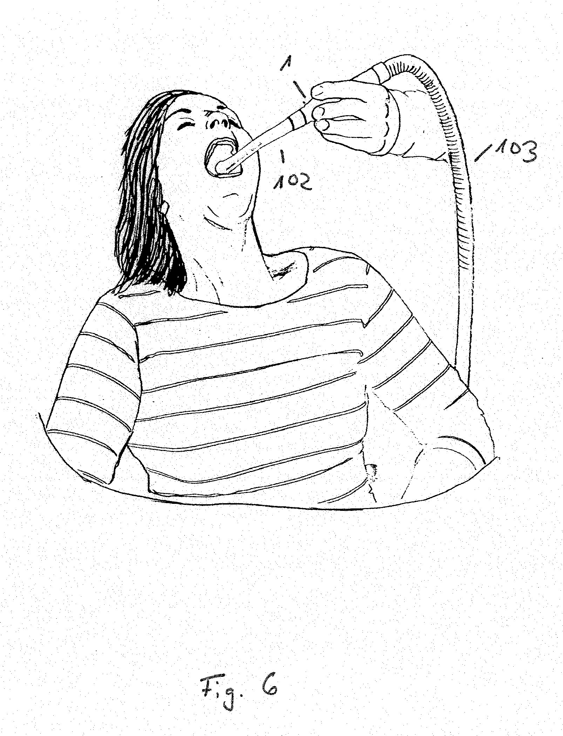

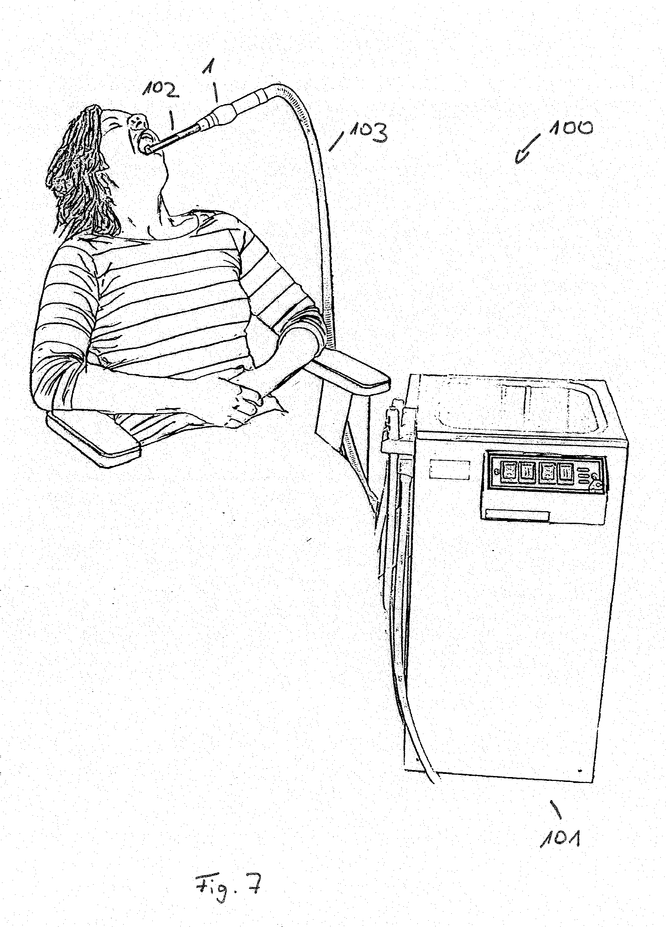

Fig. 1 zeigt ein Filtergehäuse 2 eines erfindungsgemäßen Filters 1. Das Filtergehäuse 2 ist als rohrförmiger Hohlkörper mit zwei Öffnungen 4, 6 ausgebildet, und zwar in Strömungsrichtung gerade, so dass die beiden an den Enden befindlichen Öffnungen, die erste Gehäuseöffnung 4 und die zweite Gehäuseöffnung 6, einander gegenüberliegen. Das Filtergehäuse 2 kann auch gebogen oder in sonstiger Weise ausgeführt sein. Das in Fig. 1 gezeigte Filtergehäuse 2 ist einteilig ausgebildet; auch eine mehrteilige Ausbildung ist möglich. Das Filtergehäuse 2 ist rotationssymmetrisch. Der Querschnitt (senkrecht zur Strömungsrichtung) des Filtergehäuses 2 ist kreisförmig ausgebildet, kann aber auch andere Geometrien aufweisen. Das Filtergehäuse 2 weist an der ersten Gehäuseöffnung 4 einen Schlauchanschlussabschnitt 3 und an der zweiten Gehäuseöffnung 6 einen Eingangsabschnitt 5 auf. Wie in Fig. 6 bzw. Fig. 7 zu sehen, wird das Filtergehäuse 2 an dem Schlauchanschlussabschnitt 3 mit dem einen Teil 103 der Absaugeinrichtung verbunden, an dessen anderem Ende sich der Vakuumerzeuger 101 befindet. Die Verbindung mit der Absaugeinrichtung erfolgt über Schlauch 103. An dem Eingangsabschnitt 6 wird das Filtergehäuse 2 mit dem anderen Teil der Absaugeinrichtung verbunden. Dabei handelt es sich um den Teil, an dessen anderen Ende sich das Mundstück 102 befindet. Der Eingangsabschnitt 6 ist direkt mit dem Mundstück 102 der Absaugeinrichtung 100 verbunden, und der Eingangsabschnitt 6 ist so ausgebildet, dass er auf das Mundstück 102 der Absaugeinrichtung 100 gesteckt werden kann.

Das Filtergehäuse 2 weist einen Umströmungsbereich 8 auf, der in Strömungsrichtung hinter dem Eingangsabschnitt 5 liegt und sich an diesen anschließt, wobei der Umströmungsbereich einen größeren Innendurchmesser g2 aufweist als der Eingangsabschnitt 5, der einen Innendurchmesser g1 aufweist. Der Übergang zwischen Eingangsabschnitt 5 und Umströmungsbereich 8 erfolgt unter Bildung eines Absatzes 7a. Durch den Absatz ist gewährleistet, dass der Filtersack (in Fig. 1 nicht gezeigt) im Umströmungsbereich nicht an der Filtergehäusewand anliegt, was eine Verringerung der offenen Filterfläche mit sich brächte und daher nachteilig wäre.

An seinem anderen Ende schließt sich an den Umströmungsbereich 8 der Schlauchanschlussabschnitt 3 an, der somit in Strömungsrichtung hinter dem Umströmungsbereich 8 liegt und den Umströmungsbereich 8 begrenzt.

Die Filtergehäusewand verläuft im Anschluss an den Absatz 7a nicht gerade, sondern erweitert sich konisch. Diese konische Erweiterung bewirkt, dass weniger Strömungswirbel entstehen, und vergrößert die Strömungsgeschwindigkeit kurz hinter dem Übergang von Eingangsabschnitt 5 zu Umströmungsbereich 8. Auf diese Weise wird die Absaugflüssigkeit zügiger durch den Filter gezogen. Die Saugleistung des Filters wird also besonders stark erhöht.

Bei der in der in Fig. 1 gezeigten Ausführungsform weist der Umströmungsbereich 8 zwei Abschnitte 8a, 8b auf, wobei der Übergang vom ersten Umströmungsbereichsabschnitt 8a zum zweiten Umströmungsbereichsabschnitt 8b, der in Strömungsrichtung hinter dem ersten Umströmungsbereichsabschnitt 8a angeordnet ist, ebenfalls unter Bildung eines Absatzes 7b erfolgt und der zweite Umströmungsbereichsabschnitt 8b einen größeren Innendurchmesser g3 aufweist als der erste Umströmungsbereichsabschnitt 8a. Eine solche Ausbildung ist besonders günstig, da sie nochmals verbesserte Strömungseigenschaften mit sich bringt. Auch im zweiten Umströmungsbereichsabschnitt 8b erweitert sich die Filtergehäusewand hinter dem gebildeten Absatz 7b konisch, was für die Strömungseigenschaften besonders vorteilhaft ist.

Wie vorstehend ausgeführt, kann das Filtergehäuse 2 über den Eingangsabschnitt 5 direkt mit dem Mundstück 102 der Absaugeinrichtung 100 verbunden werden.

Alternativ weist der Filter 1 zusätzlich eine Filterhülse 9 auf, wie sie in Fig. 2 gezeigt ist.

Die Filterhülse 9 ist als rohrförmiger Hohlkörper mit zwei Öffnungen 11, 13, einer ersten Hülsenöffnung 11 und einer zweiten Hülsenöffnung 13, ausgebildet, und zwar in Strömungsrichtung gerade, so dass die beiden an den Enden befindlichen Hülsenöffnungen 11, 13 einander gegenüberliegen. Die Filterhülse 9 ist einteilig ausgebildet. Sie kann jedoch auch mehrteilig ausgebildet sein. Der Querschnitt (senkrecht zur Strömungsrichtung) der Filterhülse 9 ist er kreisförmig ausgebildet. Die Filterhülse 9 weist an der ersten Hülsenöffnung 11 einen Mundstückanschlussabschnitt 10 und an der zweiten Hülsenöffnung 13 einen Gehäuseverbindungsabschnitt 12 auf. Wie in Fig. 6 und 7 zu sehen ist, wird die Filterhülse 9 und damit der Filterkörper 2, 9 an dem Mundstückanschlussabschnitt 10 mit dem Mundstück 102 der Absaugeinrichtung 100 verbunden und der Mundstückanschlussabschnitt 10 ist so ausgebildet, dass er auf das Mundstück 102 der Absaugeinrichtung 100 gesteckt werden kann.

Am Gehäuseverbindungsabschnitt 12 der Filterhülse 9 ist eine Filtersackanbringhilfe 14 vorgesehen. Diese ermöglicht es, das sackförmige Filtergewebe einfacher an dem Gehäuseverbindungsabschnitt 12 der Filterhülse 9 anzubringen. Die Filtersackanbringhilfe ist in Form von sich von dem Gehäuseverbindungsabschnitt 12 in Längsrichtung erstreckenden, am Ende nach innen zulaufenden Dornen gebildet 14. Diese erleichtern ein Öffnen des Filtersacks 15 und ein Aufschieben des Filtersacks auf den Gehäuseverbindungsabschnitt. Weiterhin unterstützen sie, dass der Filtersack offen gehalten wird. Die Dornen sind flexibel ausgebildet.

Fig. 3 zeigt die Filterhülse 9 mit über den Gehäuseverbindungsabschnitt 12 gezogenem Filtergewebesack 15. Der Filtersack 15 ist mit seiner Sacköffnung 16 über den Gehäuseverbindungsabschnitt 12 gezogen. Er weist eine Seitennaht 17 und eine Bodennaht 18 auf. Die offene Fläche des Filtergewebes beträgt das Doppelte der Fläche des Querschnitts des äußeren Endes 3a des Schlauchanschlussabschnitts 3. Das Filtergewebe besteht aus einem PET-Gewebe mit Quadratmaschen, wobei die Maschenweite w 30 μm beträgt. Der Fadendurchmesser d der Fäden des Filtergewebes ist für Kett- und Schussfaden gleich. Der Fadendurchmesser d beträgt jeweils 36 μm.

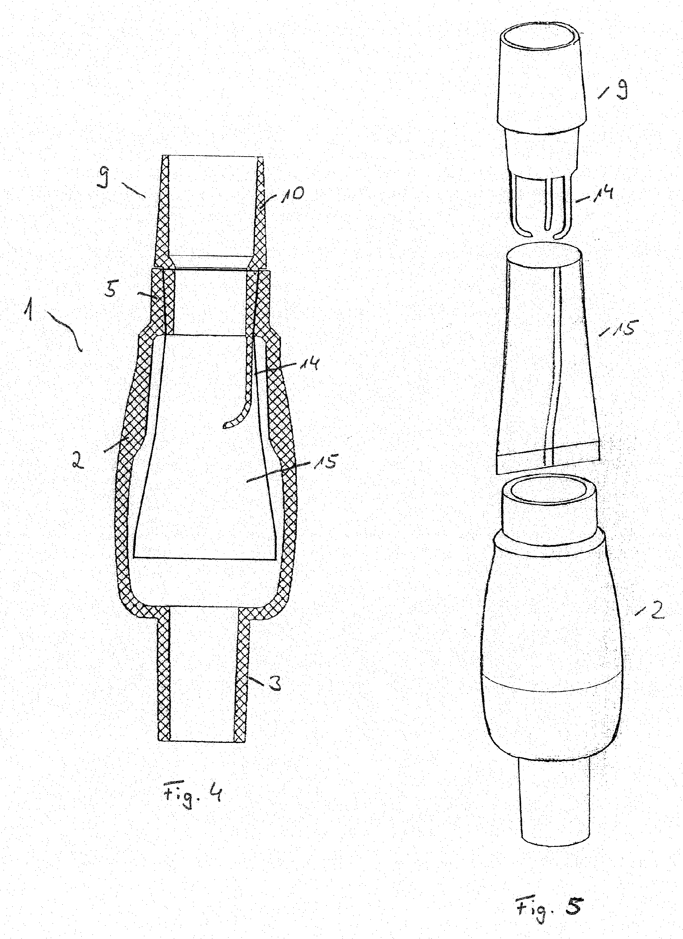

Fig. 4 zeigt den Filter 1 in montiertem, d.h. zusammengesetztem Zustand. Eingangsabschnitt 5 des Filtergehäuses 2 und Gehäuseverbindungsabschnitt 12 der Filterhülse 9 bilden im montierten Zustand die Befestigung des Filtergewebesacks 15. Die Verbindung erfolgt mittels Klemmverbindung von Eingangsabschnitt 5 und Gehäuseverbindungsabschnitt 12. Zwischen den beiden Teilen 5, 12 ist der Filtergewebesack 15 eingeklemmt.

Die Außenwand des Gehäuseverbindungsabschnittes 12 der Filterhülse 9 verjüngt sich zur ersten Hülsenöffnung 11 hin konisch, und die Innenwand des Eingangsabschnitts 5 des Filtergehäuses 2 erweitert sich dazu korrespondierend zur zweiten Gehäuseöffnung 6 hin ebenfalls konisch. Dabei ist bei direktem Zusammenstecken von Filterhülse 9 und Filtergehäuse 2 noch ein geringes Spiel vorhanden, so dass sich bei Zwischenstecken des Filtersacks 15 eine gut schließende Klemmpassung ergibt.

In Fig. 5 ist der Filter 1 in Explosionsdarstellung gezeigt. Filterhülse 9, Filtersack 15 und Filtergehäuse 2 sind gezeigt. Die Strömungsrichtung geht von der Filterhülse 9 in Richtung Filtergehäuse 2. Der Filtergewebesack 15 ist elastisch verformbar ausgebildet. Daher kann er zusammengedrückt werden, wenn er in das Filtergehäuse 2 eingeführt wird. Wenn sich der Filtergewebesack 15 im Inneren des Filtergehäuses 2 befindet, nimmt er zumindest im Wesentlichen wieder seine ursprüngliche Gestalt an.

Das Filtergehäuse weist die mit Bezug auf Fig.

1 beschriebene Gestaltung mit Absatz 7a und einer sich daran anschließenden konischen Erweiterung des Umströmungsbereiches 8 auf. Diese Ausgestaltung führt dazu, dass sich leichtere Partikel im oberen Bereich, d.h. im Bereich der Sacköffnung 16 des Filtergewebesacks 15 ablagern, während schwerere Partikel bis in den unteren (Boden)Bereich, d.h. den Bereich der Bodennaht 18 des Filtergewebesacks 15 gelangen.

Fig. 6 und 7 zeigen eine Ausführungsform des erfindungsgemäßen Filters 1 eingesetzt eine zahnärztliche Absaugeinrichtung 100. Absaugeinrichtung 100 besteht aus einem Vakuumerzeuger 101 und einem Rohr-/Schlauchsystem 103. Sie dienen dazu, bei Schleif- und Bohrarbeiten an den Zähnen eines Patienten entstehende Partikel aus dem Behandlungsbereich im Mundraum zu entfernen. Am einen Ende der Absaugeinrichtung 100 befindet sich das Mundstück 102, das in den Mund des Patienten eingeführt wird. An das Mundstück 102 schließt sich ein Schlauch 103 an, auf den das Mundstück 102 aufgesteckt ist. Am anderen Ende der Absaugeinrichtung 100 ist üblicherweise ein Amalgamfilter (nicht gezeigt) angebracht, um Amalgamreste aus der Absauglösung herauszufiltern, bevor diese ins Abwasser entlassen wird. Im Amalgamfilter werden auch weitere Feststoffpartikel abgeschieden.

Die Fig. 6 und 7 zeigen den Filter im Einsatz bei einer zahnärztlichen Behandlung. Zunächst wird der Filter vorbereitet. Dazu wird der Filtergewebesack 15 an dem Gehäuseverbindungsabschnitt 12 auf die Filterhülse 8 geschoben. Das Aufschieben wird erleichtert durch die Dornen 14. Im nächsten Schritt wird der Gehäuseverbindungsabschnitt 12 der Filterhülse 9 unter Zusammendrücken des Filtergewebesacks 15 und Zusammenfügen von Filterhülse 9 und Filtergehäuse 2 zum Filter 1 unter klemmender Befestigung des Filtergewebesacks 15 in den Eingangsabschnitt 5 des Filtergehäuses 2 eingeführt. Wenn abzusehen ist, dass im Laufe der zahnärztlichen Behandlung im Mund des Patienten edelmetallhaltige Partikel anfallen, wird der zusammengesetzte Filter 1 zwischen Mundstück 102 und Schlauch 103 in die zahnärztliche Absaugeinrichtung 100 eingefügt. Im Folgenden werden die Edelmetallpartikel mit der Absaugflüssigkeit abgesaugt. Die Edelmetallpartikel werden in dem Filtergewebesack 15 aufgefangen. Sind alle Edelmetallpartikel abgesaugt oder ist der Filter 1 voll, wird er aus der Absaugeinrichtung 100 zwischen Mundstück 102 und Schlauch 103 herausgenommen. Dabei kann wahlweise entweder nur die Filterhülse 8 mit dem Filtergewebesack 15 entnommen werden oder aber der gesamte Filter 1 wird einschließlich des Filtergehäuses 2 entnommen. Die erstere Variante kommt insbesondere dann in Frage, wenn noch nicht alle Edelmetallpartikel aufgesaugt wurden. Wenn alle Edelmetallpartikel jedoch aufgesaugt sind, wird zumeist der gesamte Filter entnommen.

Vor der nächsten Verwendung werden die Filterteile 2, 9 sterilisiert. Auch der Filtergewebesack wird üblicherweise sterilisiert. Die Edelmetallpartikel sind im Filtergewebesack 15 enthalten. Aufgrund der Sackform sind die Partikel geschützt und die Gefahr eines Verlustes ist gering. Der Filtergewebesack 15 wird mit den darin enthaltenen Edelmetallpartikeln einem Sammelbehälter zugeführt. Dem Sammelbehälter werden weitere Filtergewebesäcke 15 zugeführt, bis eine gewünschte Menge erreicht ist. Dann wird der Sammelbehälter zu einer Recyclingeinrichtung gebracht. Dort wird das Edelmetall durch Aufschmelzen der Edelmetallpartikel wiedergewonnen.

Bezugszeichenliste

| | |
|---|---|
| 1 | Filter |
| 2 | Filtergehäuse |
| 3 | Schlauchanschlussabschnitt |
| 3a | äußeres Ende des Schlauchanschlussabschnitts |
| 4 | Erste Gehäuseöffnung |
| 5 | Eingangsabschnitt |
| 6 | Zweite Gehäuseöffnung |
| 7a | Erster Absatz |
| 7b | zweiter Absatz |
| 8 | Umströmungsbereich |
| 8a | erster Umströmungsbereichsabschnitt |
| 8b | zweiter Umströmungsbereichsabschnitt |
| 9 | Filterhülse |
| 10 | Mundstückanschlussabschnitt |
| 11 | Erste Hülsenöffnung |
| 12 | Gehäuseverbindungsabschnitt |
| 13 | Zweite Hülsenöffnung |
| 14 | Filtersackanbringhilfe |
| 15 | Filtergewebe(sack) |
| 16 | Sacköffnung |
| 17 | Seitennaht |
| 18 | Bodennaht |
| | |
| 100 | Absaugeinrichtung |
| 101 | Vakuumerzeuger |
| 102 | Mundstück |
| 103 | Schlauch |

**Patentansprüche**

1. Filter (1), ausgebildet und eingerichtet zum Einfügen in eine Absaugeinrichtung (100), umfassend
   ein Filtergewebe (15) und
   einen Filterkörper zur Aufnahme des Filtergewebes (15), wobei der Filterkörper ein als Hohlkörper mit mindestens zwei Öffnungen (4, 6) ausgebildetes Filtergehäuse (2) mit einem Schlauchanschlussabschnitt (3) an einer ersten Gehäuseöffnung (4) und mit einem Eingangsabschnitt (5) an einer zweiten Gehäuseöffnung (6) umfasst;
   **dadurch gekennzeichnet, dass**
   das Filtergewebe (15) sackförmig ausgebildet ist,

dass die offene Fläche des Filtergewebes (15) mindestens das Doppelte der Fläche des Querschnitts des äußeren Endes des Schlauchanschlussabschnitts (3) beträgt,
dass das Filtergewebe (15) Abschnitte mit einer Maschenweite w von 10 bis 80 μm umfasst und
dass der Fadendurchmesser d der Fäden dieser Abschnitte des Filtergewebes (15) von 10 bis 80 μm beträgt.

2. Filter (1) nach Anspruch 1, wobei der Filter (1) zum Einfügen in eine ärztliche, insbesondere eine zahnärztliche, Absaugvorrichtung (100) ausgebildet und eingerichtet ist.

3. Filter (1) nach Anspruch 1 oder 2, **dadurch gekennzeichnet, dass** der Filterkörper des Weiteren eine als Hohlkörper mit mindestens zwei Öffnungen (11, 13) ausgebildete Filterhülse (9) mit einem Mundstückanschlussabschnitt (10) an einer ersten Hülsenöffnung (11) und mit einem Gehäuseverbindungsabschnitt (12), der zur Verbindung mit dem Eingangsabschnitt (5) des Gehäuses (2) ausgebildet ist, an einer zweiten Hülsenöffnung (13) umfasst.

4. Filter (1) nach einem der Ansprüche 1 bis 3, **dadurch gekennzeichnet, dass** der Eingangsabschnitt (5) des Gehäuses (2) und der Gehäuseverbindungsabschnitt (12) der Filterhülse (9) im montierten Zustand die Befestigung des Filtersackes (15) bilden.

5. Filter (1) nach Anspruch 3 oder 4, **dadurch gekennzeichnet, dass** die Filterhülse (9) als rohrförmiger gerader Hohlkörper mit einander gegenüberliegender erster und zweiter Hülsenöffnung (11, 13) ausgebildet ist.

6. Filter (1) nach einem der Ansprüche 1 bis 5, **dadurch gekennzeichnet, dass** das Filtergehäuse (2) als rohrförmiger gerader Hohlkörper mit einander gegenüberliegender erster und zweiter Gehäuseöffnung (4, 6) ausgebildet ist.

7. Filter (1) nach einem der Ansprüche 1 bis 6, **dadurch gekennzeichnet, dass** der Eingangsabschnitt (5) des Filtergehäuses (2) sich zur zweiten Gehäuseöffnung (6) hin konisch erweiternd ausgebildet ist.

8. Filter (1) nach Anspruch 7, **dadurch gekennzeichnet, dass** der Gehäuseverbindungsabschnitt (12) der Filterhülse (9) sich zur zweiten Hülsenöffnung (13) hin konisch verjüngend ausgebildet ist.

9. Filter (1) nach einem der Ansprüche 3 bis 8, **dadurch gekennzeichnet, dass** am Gehäuseverbindungsabschnitt (12) der Filterhülse (9) eine Filtersackanbringhilfe (14) vorgesehen ist.

10. Filter (1) nach einem der Ansprüche 1 bis 9, **dadurch gekennzeichnet, dass** das Filtergehäuse (2) des Weiteren einen Umströmungsbereich (8) aufweist, der in Strömungsrichtung hinter dem Eingangsabschnitt (5) liegt und der einen größeren Innendurchmesser aufweist als der Eingangsabschnitt (5), wobei der Übergang zwischen Eingangsabschnitt (5) und Umströmungsbereich (8) unter Bildung eines Absatzes erfolgt.

11. Filter (1) nach Anspruch 10, **dadurch gekennzeichnet, dass** die Filtergehäusewand im Anschluss an den Absatz (7a) sich konisch erweiternd ausgebildet ist.

12. Filter (1) nach Anspruch 10 oder 11, **dadurch gekennzeichnet, dass** sich der eingesetzte Filtersack (15) über zwischen 70% und 95%, vorzugsweise über zwischen 75% und 90% und insbesondere zwischen 80% und 95% der Länge des Umströmungsbereichs (8) erstreckt.

13. Filter (1) nach einem der Ansprüche 1 bis 12, **dadurch gekennzeichnet, dass** das Filtergehäuse (2) und/oder die Filterhülse (9) rotationssymmetrisch ausgebildet sind.

14. Filter (1) nach einem der Ansprüche 1 bis 13, **dadurch gekennzeichnet, dass** Filtergewebe (15) autoklavenbeständig ist.

15. Zahnärztliche Absaugeinrichtung (100), umfassend einen Vakuumerzeuger (101), ein Rohr-/ Schlauchsystem (103) und ein Mundstück (102), **dadurch gekennzeichnet, dass** sie weiterhin einen Filter (1) nach einem der Ansprüche 1 bis 14 umfasst.

16. Filterhülse (9) als Einführhilfe für einen Filtersack (15) in einen Filter (1) einer Absaugeinrichtung (100), insbesondere zur Verwendung in einem Filter (1) nach einem der Ansprüche 1 bis 14, wobei die Filterhülse (9) als Hohlkörper mit mindestens zwei Öffnungen ausgebildet (11, 13) ist, wobei sich an einer ersten der mindestens zwei Öffnungen ein Mundstückanschlussabschnitt (10) zur Verbindung der Hülse (9) mit einer Seite der Absaugeinrichtung (100) befindet und sich an einer zweiten der mindestens zwei Öffnungen ein Filteranbringabschnitt (12) befindet, an dem der Filtersack (15) an der Filterhülse (9) angebracht wird, **dadurch gekennzeichnet, dass** die Filterhülse (9) am Filteranbringabschnitt (12) eine Filtersackanbringhilfe (14) aufweist und der Filteranbringabschnitt (12) zur Verbindung mit dem Filter (1) ausgebildet ist.

17. Verfahren zum Gewinnen und Wiederverwerten von Edelmetallen, insbesondere von Gold oder goldhaltigen Legierungen, aus zahnärztlichen Absauglösungen, insbesondere unter Verwendung eines Filters (1) nach einem der Ansprüche 1 bis 14, umfassend die folgenden Schritte:

a) Aufziehen eines Filtergewebesackes (15) auf eine Filterhülse (9) auf einen Gehäuseverbindungsabschnitt (12);
b) Einführen des Gehäuseverbindungsabschnitts (12) der Filterhülse (9) in einen Eingangsabschnitt (5) eines Filtergehäuses (2) unter Zusammendrücken des Filtergewebesacks (15) und Zusammenfügen von Filterhülse (9) und Filtergehäuse (2) zum Filter (1) unter klemmender Befestigung des Filtergewebesacks (15);
c) Einfügen des zusammengesetzten Filters (1) in eine zahnärztliche Absaugeinrichtung (100) zwischen Mundstück (102) und Schlauch (103);
d) Absaugen von Edelmetallpartikel enthaltender Absaugflüssigkeit;
e) Auffangen der Edelmetallpartikel in dem Filtergewebesack (15);
f) Herausnehmen des Filters (1) aus der Absaugeinrichtung zwischen Mundstück (102) und Schlauch(103).

18. Verfahren nach Anspruch 17, weiterhin umfassend die Schritte

g) Sterilisieren mindestens eines der Teile Filtergehäuse (2), Filterhülse (9) und Filtergewebesack (15);
h) Zuführen des Edelmetallpartikel enthaltenden Filtergewebesacks (15) zu einem Sammelbehälter.

19. Verfahren nach Anspruch 18, weiterhin umfassend die Schritte

i) Transport des Sammelbehälters nach Erreichen einer gewünschten gesammelten Menge an Edelmetallpartikel enthaltenden Filtergewebesäcken (15) zu einer Recyclingeinrichtung;
j) Wiedergewinnen von Edelmetall aus den Edelmetallpartikeln durch Aufschmelzen in der Recyclingeinrichtung.

Fig. 1

Fig. 2

Fig. 3

Fig. 4

Fig. 5

EP 2 929 857 A1

Fig. 6

17

Fig. 7

Europäisches
Patentamt

European
Patent Office

Office européen
des brevets

**EUROPÄISCHER RECHERCHENBERICHT**

Nummer der Anmeldung

EP 14 16 4356

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 4 083 706 A (WILEY CORLESS W) 11. April 1978 (1978-04-11) | 16-19 | INV. A61C17/06 A61M1/00 |
| Y | * Spalte 1 - Spalte 6; Abbildungen 1-3 * | 1-9, 13-15 | |
| A | | 10-12 | |
| | ----- | | |
| A | US 6 299 763 B1 (ASHMAN ARTHUR [US]) 9. Oktober 2001 (2001-10-09) * Spalten 1, 4, 5 * | 1-19 | |
| | ----- | | |
| Y | US 3 701 433 A (KRAKAUER SIDNEY ET AL) 31. Oktober 1972 (1972-10-31) | 1-9, 13-15 | |
| A | * Spalte 2, Zeile 22 - Zeile 63; Anspruch 4; Abbildung 4 * | 10-12, 16-19 | |
| | ----- | | |

| RECHERCHIERTE SACHGEBIETE (IPC) |
|---|
| A61C A61M |

Der vorliegende Recherchenbericht wurde für alle Patentansprüche erstellt

| Recherchenort | Abschlußdatum der Recherche | Prüfer |
|---|---|---|
| München | 16. Oktober 2014 | Wirth, Christian |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 14 16 4356

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-10-2014

| Im Recherchenbericht angeführtes Patentdokument | Datum der Veröffentlichung | Mitglied(er) der Patentfamilie | Datum der Veröffentlichung |
|---|---|---|---|
| US 4083706 A | 11-04-1978 | KEINE | |
| US 6299763 B1 | 09-10-2001 | KEINE | |
| US 3701433 A | 31-10-1972 | AU 463008 B2 | 10-07-1975 |
| | | AU 3549771 A | 17-05-1973 |
| | | BE 775135 A1 | 10-05-1972 |
| | | CA 968720 A1 | 03-06-1975 |
| | | DE 2155820 A1 | 22-06-1972 |
| | | DK 133366 B | 10-05-1976 |
| | | FI 52274 B | 02-05-1977 |
| | | FR 2113884 A1 | 30-06-1972 |
| | | GB 1334555 A | 24-10-1973 |
| | | IT 944837 B | 20-04-1973 |
| | | JP S5532383 B1 | 25-08-1980 |
| | | NL 7115463 A | 15-05-1972 |
| | | NO 128592 B | 17-12-1973 |
| | | SE 386832 B | 23-08-1976 |
| | | US 3701433 A | 31-10-1972 |
| | | ZA 7107534 A | 30-08-1972 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE G8314829
- DE 102009034143 A
- DE 541402
- EP 1432381 B1